# EUROPEAN PATENT SPECIFICATION

(11) **EP 1 551 838 B1**
(45) Date of publication and mention of the grant of the patent: **09.08.2006**
(21) Application number: 03809127.8
(22) Date of filing: 16.10.2003
(51) Int. Cl.: C07D 473/18

(54) **METHOD FOR REDUCING RESIDUAL ALCOHOLS IN CRYSTALLINE VALACYCLOVIR HYDROCHLORIDE**
VERFAHREN ZUR VERMINDERUNG VON RESTALKOHOLEN IM KRISTALLINEN VALACYCLOVIR-HYDROCHLORID
PROCEDE POUR REDUIRE LA QUANTITE D'ALCOOLS RESIDUELS DANS DE L'HYDROCHLORURE DE VALACYCLOVIR CRISTALLIN

(30) Priority: 16.10.2002 US 419270 P; 18.11.2002 US 427320 P
(43) Date of publication of application: 13.07.2005
(73) Proprietor: TEVA PHARMACEUTICAL INDUSTRIES LTD., 49131 Petah Tiqva (IL)
(72) Inventor: DOLITZKY, Ben-Zion, Petach Tiqva 49651 (IL); LIFSHITZ, Igor, Petach-Tiqva (IL)
(74) Representative: Clyde-Watson, Zöe
(86) International application number: PCT/US2003/032995
(87) International publication number: WO 2004/035583

(56) References cited:
- EP-A- 0 665 229
- WO-A-03/041647
- US-A- 4 957 924

## Description

### BACKGROUND OF THE INVENTION

Valacyclovir is an L-valyl ester prodrug of acyclovir. Acyclovir is an acyclic analog of a natural nucleoside which has been found to have high anti-viral activity. Acyclovir is widely used in the treatment and prophylaxis of viral infections in humans, particularly infections caused by the herpes group of viruses. *See* Goodman and Gilman's, *The Pharmacological Basis of Therapeutics* 1193-1198 (9th ed. 1996).

Acyclovir is an acyclic guanine nucleoside analog that lacks a 3'-hydroxyl on the side chain. Acyclovir has the chemical name 6H-Purin-6-one, 2-amino-1,9-dihydro-9-[(2-hydroxyethoxy)methyl]. (CAS Registry No. 59277-89-3.) Acyclovir as the sodium salt is currently marketed as ZOVIRAX® .

Valacyclovir has the chemical name 1-valine, 2-[(2-amino-1,6-dihydro-6-oxo -9H-purin-9-yl)methoxy]ethyl ester. (CAS Registry No. 124832-26-4.) Valacyclovir is currently marketed as VALTREX® . The chemical structure of valacyclovir is shown as Formula I.

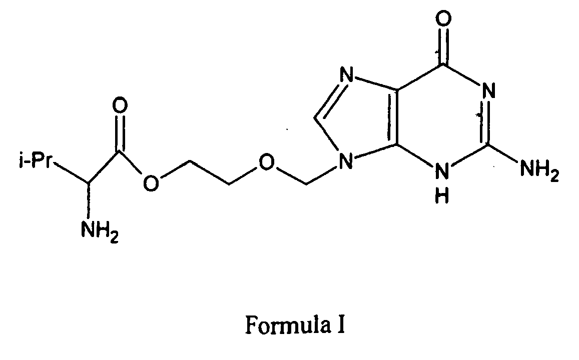

For oral administration, it is advantageous to administer valacyclovir rather than acyclovir because acyclovir is poorly absorbed from the gastrointestinal tract after oral administration in both animals and humans. In contrast, valacyclovir is rapidly absorbed from the gastrointestinal tract after oral administration. Moreover, valacyclovir is converted rapidly and virtually completely to acyclovir after oral administration in healthy adults. The conversion of valacyclovir is thought to result from first-pass intestinal and hepatic metabolism through enzymatic hydrolysis.

Work-up, isolation, and purification procedures for valacyclovir hydrochloride can and frequently do use solvents that are or that contain alcohols such as methanol, ethanol or *iso*-propanol. United States Patent 4,957,924 discloses one such crystallization procedure that uses ethanol. In such cases, when alcohols are used in work-up or other procedures, the valacyclovir hydrochloride can contain 5000 ppm or more of excess residual process alcohol. The presence of unnecessary foreign substances, for example excess residual process alcohols, in any active pharmaceutical ingredient (API) is undesirable. These excess residual process alcohols are not necessary to the efficacy of the API valacyclovir hydrochloride. The solvents may be toxic and can produce undesirable effects in the patient receiving valacyclovir hydrochloride. Since there is no therapeutic benefit from residual process solvents, all residual solvents should be removed to the extent possible to meet quality-based requirements.

Indeed, health regulatory agencies in many countries have established limits for foreign substances in active pharmaceutical ingredients and may require manufacturers to adapt manufacturing procedures to reduce or eliminate them. For example, the United States Food and Drug Administration has promulgated guidelines (Q3C) that apply to residual solvents in drug substances and drug products.

The International Conference on Harmonization of Technical Requirements for Registration of Pharmaceuticals for Human Use has also promulgated Draft Guidelines (Q3C) for Residual Solvents in Pharmaceuticals. *See* Step 4 Draft, July 16, 1997, Dr. Shigeo Kojima, rapporteur (hereafter ICH Guidelines). The draft proposes three classes of solvents and several options for quantifying the permissible level of them. Class 3 solvents should be limited (Option 1) to 5000 ppm, provided that the total daily dosage would be less than 50 mg (concentration in tablet should not exceed 5000 ppm). Ethanol and the propanols are among the class 3 solvents that should be limited by good manufacturing procedures (GMP).

Although some residual process solvent in an API or drug product may be an unavoidable consequence of the manufacturing process, the level of residual process solvent should be reduced to a minimum. Clearly, methods for reducing excess process solvents like alcohols in valacyclovir hydrochloride to a level less than 5000 ppm are needed.

### SUMMARY OF THE INVENTION

In one aspect, the present invention relates to a method of reducing excess residual process alcohol (> 5000 ppm) in valacyclovir hydrochloride having excess residual process alcohol, including the step of statically or dynamically contacting the valacyclovir hydrochloride having excess residual process alcohol with a humid gas, especially humid air.

In another aspect, the present invention relates to a method of reducing excess residual process alcohol, especially excess residual process isopropanol, in valacyclovir hydrochloride having excess residual process alcohol including the step of dynamically contacting such valacyclovir hydrochloride with humid air, especially humid air of ≥50% relative humidity, more especially ≥75% relative humidity, in a fluiduzed bed apparatus.

### DETAILED DESCRIPTION OF THE INVENTION

One skilled in the art of the synthesis of organic compounds understands that solvents, e.g. alcohols, are often used in synthesis procedures and that traces, sometimes substantial traces, of these solvents can remain in the compound synthesized. The remaining solvents, which can be referred to as residual process solvents, can be difficult to remove.

Residual process solvents in pharmaceutical compounds (and ultimately any pharmaceutical compositions prepared therefrom) serve no therapeutic purpose and can be harmful to the patient. As discussed above, governmental regulatory agencies and international advisory organizations have promulgated regulations and guidelines for residual (process) solvents in pharmaceutical compounds. Excess residual process solvent in valacyclovir hydrochloride is defined in relation to the concentration limits set for class 3 solvents, of which *iso*-propanol is on example, by the ICH Guidelines. Accordingly, excess process alcohol in valacyclovir hydrochloride refers to process alcohol, especially ethanol and iso-propanol, in excess of 5000 ppm on a weight basis. Valacylcovir hydrochloride having excess residual process alcohol refers to valacyclovir hydrochloride having 5000 ppm or more, on a weight basis, residual process alcohol.

Alcohols can be used as solvents in the synthesis, work-up, and purification of valacyclovir hydrochloride. The present invention provides a method for reducing the excess residual process alcohol content of crystalline valacyclovir hydrochloride having excess residual process alcohols, for example ethanol, n-propanol, or *iso*-propanol, remaining from, for example, work-up, isolation, or other treatment procedures, for example recrystallization. Valacyclovir hydrochloride is considered to have excess residual process alcohol if the residual process alcohol is ≥ 5000 ppm on a weight basis. The present method includes the step of contacting (exposing) particles (e.g. crystals) of valacyclovir hydrochloride having excess residual process alcohol with a humid gas, at ambient atmospheric pressure (750 to 765 mm Hg).

The valacyclovir hydrochloride having excess residual process alcohol or process alcohol (residual process alcohol of 5000 ppm or more) can be from any source. Typically, the valacyclovir hydrochloride will be obtained from a process in which an alcohol or alcohol-containing solvent is used, for example from a crystallization procedure in which an alcohol is used. But valacyclovir hydrochloride having excess residual process alcohol can be obtained directly from a synthesis process in which an alcohol is used. In such cases, the excess residual process alcohol can be more than 5000 ppm.

Any gas that does not induce or accelerate chemical degradation of valacyclovir hydrochloride during the treatment process can be used. Air is the preferred gas. Humid gas has a relative humidity (RH) of at least 15%, preferably at least 50%, more preferably at least 75%. Relative humidity refers to the ratio (times 100) of the actual vapor pressure of water in a gas to the saturation vapor pressure of water in the gas at a particular temperature and pressure.

The contacting is conducted at ambient pressure and a temperature of 10°C to 60°C. The contacting can be static or it can be dynamic.

In static contacting, particles (e.g. crystals) of valacyclovir hydrochloride are at rest. That is, they are not mechanically or otherwise agitated or stirred. Static contacting can be carried out, for example, by contacting particles of valacyclovir hydrochloride having excess residual process alcohol on a tray, preferably in a thin layer, with humid gas in a suitable enclosure such as, for example, a constant humidity chamber.

In dynamic contacting, particles of valacyclovir hydrochloride are in motion induced by mechanical or other agitation whilst being contacted with humid gas. Mechanical agitation can be provided by, for example, a ribbon-type blender through which humid gas is passed.

In a preferred embodiment, the valacyclovir hydrochloride having excess residual process alcohol is contacted with humid gas in a fluidized bed apparatus wherein the valacyclovir hydrochloride is fluidized with humid gas. Fluidized bed apparatus is well-known in the art. One example of suitable fluidized bed apparatus is a Retsch model TG-100

Valacyclovir hydrochloride having excess residual process alcohol is contacted with humid air for a contacting time sufficient to reduce the excess residual process alcohol to less that 5000 ppm, preferably to 1000 ppm or less. The skilled artisan will know to optimize the contacting time by routine experimentation, taking into consideration factors such as the amount of excess residual process alcohol initially present, the size of the particles of valacyclovir hydrochloride, and the humidity of the humid gas. The higher the initial amount of excess residual process alcohol, the larger the particles of valacyclovir hydrochloride, and the lower the humidity of the humid gas, the longer will be, in general, the contacting time. The lower the initial amount of excess residual process alcohol and the higher the humidity of the humid gas, the shorter, in general, will be the contacting time.

By the method of the present invention, the excess residual process alcohol in valacyclovir hydrochloride is reduced to < 5000 ppm, preferably to 1000 ppm or less. Alcohol in valacyclovir hydrochloride can be measured by any means known in the art, for example by gas chromatography (GC).

The present invention can be illustrated with the following nonlimiting examples.

### Example 1

Valacyclovir hydrochloride (about 10g), crystallized from isopropanol/water and having about 6000 ppm excess residual process solvent, were dried in a fluidized bed drier at about 40°C for about 4 hours in a stream of humid air (ca. 80% RH). After drying, the material so treated contained less than about 300 ppm residual process solvent and about 9% by weight water.

## Claims

1. A method for reducing excess residual process alcohol in valacyclovir hydrochloride having excess residual process alcohol comprising the step of contacting particles of valacyclovir hydrochloride having excess residual process alcohol with a humid gas at ambient pressure.

2. The method of claim 1 wherein the humid gas is humid air.

3. The method of claim 2 wherein the humid air has a relative humidity of at least 15%.

4. The method of claim 3 wherein the humid air has a relative humidity of at least 50%.

5. The method of claim 4 wherein the humid air has a relative humidity of at least 75%.

6. The method of claim 1 wherein the contacting is static.

7. The method of claim 1 wherein the contacting is dynamic.

8. The method of claim 7 wherein the contacting is in a fluidized bed apparatus.

9. The method of claim 1 wherein, after the contacting step, the residual process alcohol in the contacted valacyclovir hydrochloride is less than 5000 ppm on a weight basis.

10. The method of claim 9 wherein the residual process alcohol in the contacted valacyclovir hydrochloride is 1000 ppm or less on a weight basis.

11. The method of claim 1 wherein the process alcohol is ethanol.

12. The method of claim 1 wherein the process alcohol is *iso*-propanol.

13. The method of claim 1 wherein the process alcohol is *iso*-propanol and wherein said method comprises the step of contacting valacyclovir hydrochloride having excess residual process *iso*-propanol with humid air at ambient pressure in a fluidized bed apparatus, wherein the relative humidity of the air is at least 15%.

14. The method of claim 13 wherein the relative humidity of the air is at least 50%.

15. The method of claim 3 wherein the residual *iso*-propanol in the contacted valacyclovir hydrochloride is 5000 ppm or less on a weight basis.

16. The method of claim 15 wherein the residual *iso*-propanol in the contacted valacyclovir hydrochloride is 1000 ppm or less on a weight basis.

17. The method of claim 1 for reducing to 1000 ppm on a weight basis or less the residual process alcohol in valacyclovir hydrochloride having greater than 1000 ppm on a weight basis residual process alcohol comprising the step of contacting, at ambient pressure, the valacyclovir hydrochloride having greater than 1000 ppm on a weight basis residual process alcohol with humid gas having a relative humidity of at least 15%.

18. The method of claim 17 wherein the humid gas has a relative humidity of at least 50%.

19. The method of claim 18 wherein the contacting is in a fluidized bed apparatus.

## Patentansprüche

1. Verfahren zum Reduzieren von überschüssigem restlichem Prozeßalkohol in Valacyclovir-Hydrochlorid mit überschüssigem restlichem Prozeßalkohol, welches die Stufe umfaßt, bei der man Partikel von Valacyclovir-Hydrochlorid mit überschüssigem restlichem Prozeßalkohol bei Umgebungsdruck mit einem feuchten Gas in Kontakt bringt.

2. Verfahren nach Anspruch 1, wobei das feuchte Gas feuchte Luft ist.

3. Verfahren nach Anspruch 2, wobei die feuchte Luft eine relative Feuchte von wenigstens 15% hat.

4. Verfahren nach Anspruch 3, wobei die feuchte Luft eine relative Feuchte von wenigstens 50% hat.

5. Verfahren nach Anspruch 4, wobei die feuchte Luft eine relative Feuchte von wenigstens 75% hat.

6. Verfahren nach Anspruch 1, wobei das Inkontaktbringen statisch erfolgt.

7. Verfahren nach Anspruch 1, wobei das Inkontaktbringen dynamisch erfolgt.

8. Verfahren nach Anspruch 7, wobei das Inkontaktbringen in einem Wirbelschichtapparat stattfindet.

9. Verfahren nach Anspruch 1, wobei nach der Stufe des Inkontaktbringens die Menge an restlichem Prozeßalkohol in dem kontaktierten Valacyclovir-Hydrochlorid auf Basis des Gewichts weniger als 5000 ppm beträgt.

10. Verfahren nach Anspruch 9, wobei die Menge an restlichem Prozeßalkohol in dem kontaktierten Valacyclovir-Hydrochlorid auf Basis des Gewichts 1000 ppm oder weniger beträgt.

11. Verfahren nach Anspruch 1, wobei der Prozeßalkohol Ethanol ist.

12. Verfahren nach Anspruch 1, wobei der Prozeßalkohol Isopropanol ist.

13. Verfahren nach Anspruch 1, wobei der Prozeßalkohol Isopropanol ist und wobei das Verfahren die Stufe umfaßt, bei der man Valacyclovir-Hydrochlorid mit überschüssigem restlichem Prozeß-Isopropanol bei Umgebungsdruck in einem Wirbelschichtapparat mit feuchter Luft in Kontakt bringt, wobei die relative Feuchte der Luft wenigstens 15% beträgt.

14. Verfahren nach Anspruch 13, wobei die relative Feuchte der Luft wenigstens 50% beträgt.

15. Verfahren nach Anspruch 13, wobei die Menge an restlichem Isopropanol in dem kontaktierten Valacyclovir-Hydrochlorid auf Basis des Gewichts 5000 ppm oder weniger beträgt.

16. Verfahren nach Anspruch 15, wobei die Menge an restlichem Isopropanol in dem kontaktierten Valacyclovir-Hydrochlorid auf Basis des Gewichts 1000 ppm oder weniger beträgt.

17. Verfahren nach Anspruch 1 zum Reduzieren der Menge an restlichem Prozeßalkohol in Valacyclovir-Hydrochlorid, welches auf Basis des Gewichts mehr als 1000 ppm an restlichem Prozeßalkohol enthält, auf 1000 ppm oder weniger auf Basis des Gewichts, wobei das Verfahren die Stufe umfaßt, bei der man das Valacyclovir-Hydrochlorid mit mehr als 1000 ppm restlichem Prozeßalkohol auf Basis des Gewichts bei Umgebungsdruck mit feuchtem Gas, welches eine relative Feuchte von wenigstens 15% hat, in Kontakt bringt.

18. Verfahren nach Anspruch 17, wobei das feuchte Gas eine relative Feuchte von wenigstens 50% hat.

19. Verfahren nach Anspruch 18, wobei das Inkontaktbringen in einem Wirbelschichtapparat stattfindet.

## Revendications

1. Procédé pour abaisser l'alcool imprégné résiduel excessif dans du chlorhydrate de valacyclovir, ayant de l'alcool imprégné résiduel excessif, comprenant l'étape de mise en contact des particules de chlorhydrate de valacyclovir ayant de l'alcool imprégné résiduel excessif avec un gaz humide à la pression ambiante.

2. Procédé selon la revendication 1, dans lequel le gaz humide est de l'air humide.

3. Procédé selon la revendication 2, dans lequel l'air humide a une humidité relative d'au moins 15%.

4. Procédé selon la revendication 3, dans lequel l'air humide a une humidité relative d'au moins 50%.

5. Procédé selon la revendication 4, dans lequel l'air humide a une humidité relative d'au moins 75%.

6. Procédé selon la revendication 1, dans lequel la mise en contact est statique.

7. Procédé selon la revendication 1, dans lequel la mise en contact est dynamique.

8. Procédé selon la revendication 7, dans lequel la mise en contact a lieu dans un appareil à lit fluidisé.

9. Procédé selon la revendication 1, dans lequel, après l'étape de mise en contact, l'alcool imprégné résiduel dans le chlorhydrate de valacyclovir après mise en contact est inférieur à 5000 ppm sur une base pondérale.

10. Procédé selon la revendication 9, dans lequel l'alcool imprégné résiduel dans le chlorhydrate de valacyclovir après mise en contact est de 1000 ppm ou moins sur une base pondérale.

11. Procédé selon la revendication 1, dans lequel l'alcool imprégné est l'éthanol.

12. Procédé selon la revendication 1, dans lequel l'alcool imprégné est l'isopropanol.

13. Procédé selon la revendication 1, dans lequel l'alcool imprégné est l'isopropanol et dans lequel ledit procédé comprend l'étape de mise en contact du chlorhydrate de valacyclovir ayant de l'isopropanol imprégné résiduel excessif avec de l'air humide à la pression ambiante dans un appareil à lit fluidisé, dans lequel l'humidité relative de l'air est d'au moins 15%.

14. Procédé selon la revendication 13, dans lequel l'humidité relative de l'air est d'au moins 50%.

15. Procédé selon la revendication 13, dans lequel l'isopropanol résiduel dans le chlorhydrate de valacyclovir après mise en contact est de 5000 ppm ou moins sur une base pondérale.

16. Procédé selon la revendication 13, dans lequel l'isopropanol résiduel dans le chlorhydrate de valacyclovir après mise en contact est de 1000 ppm ou moins sur une base pondérale.

17. Procédé selon la revendication 1 pour abaisser à 1000 ppm ou moins sur une base pondérale l'alcool imprégné résiduel dans du chlorhydrate de valacyclovir ayant plus de 1000 ppm sur une base pondérale d'alcool imprégné résiduel comprenant l'étape de mise en contact à la pression ambiante, du chlorhydrate de valacyclovir ayant plus de 1000 ppm sur une base pondérale d'alcool imprégné résiduel avec un gaz humide ayant une humidité relative d'au moins 15%.

18. Procédé selon la revendication 17, dans lequel le gaz humide a une humidité relative d'au moins 50%.

19. Procédé selon la revendication 18, dans lequel la mise en contact a lieu dans un appareil à lit fluidisé.
